(19) **Europäisches Patentamt**
**European Patent Office**
**Office européen des brevets**

(11) **EP 1 743 037 B1**

(12) **EUROPÄISCHE PATENTSCHRIFT**

(45) Veröffentlichungstag und Bekanntmachung des Hinweises auf die Patenterteilung:
**10.10.2012 Patentblatt 2012/41**

(21) Anmeldenummer: **05745259.1**

(22) Anmeldetag: **29.04.2005**

(51) Int Cl.:
**C12Q 1/68** (2006.01)

(86) Internationale Anmeldenummer:
**PCT/EP2005/004604**

(87) Internationale Veröffentlichungsnummer:
**WO 2005/108602 (17.11.2005 Gazette 2005/46)**

(54) **VERFAHREN ZUR ISOLIERUNG VON SPEZIFISCHEN MOLEKÜLEN, DIE HOCHSELEKTIV AN AUSGEWÄHLTE ZIELMOLEKÜLE BINDEN**

METHOD FOR THE ISOLATION OF SPECIFIC MOLECULES WHICH BIND IN A HIGHLY SELECTIVE MANNER TO SELECTED TARGET MOLECULES

PROCEDE POUR ISOLER DES MOLECULES SPECIFIQUES QUI SE FIXENT DE MANIERE HAUTEMENT SELECTIVE SUR DES MOLECULES CIBLES SELECTIONNEES

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **30.04.2004 DE 102004021707**

(43) Veröffentlichungstag der Anmeldung:
**17.01.2007 Patentblatt 2007/03**

(73) Patentinhaber: **CytoTools AG**
**64285 Darmstadt (DE)**

(72) Erfinder:
• **KLOCK, Gerd**
**64807 Dieburg (DE)**
• **KAISER, Dirk**
**64859 Eppertshausen (DE)**
• **FREYBERG, Mark, Andre**
**64287 Darmstadt (DE)**

(74) Vertreter: **Schultheiss, Jürgen et al**
**Patentanwalt**
**Postfach 24 01 30**
**55045 Mainz (DE)**

(56) Entgegenhaltungen:
WO-A-96/34875     US-A- 5 670 637
US-A- 6 001 988     US-B1- 6 329 145

• BRIDONNEAU P ET AL: "SITE-DIRECTED SELECTION OF OLIGONUCLEOTIDE ANTAGONISTS BY COMPETITIVE ELUTION" ANTISENSE & NUCLEIC ACID DRUG DEVELOPMENT, MARY ANN LIEBERT, INC., NEW YORK, US, Bd. 9, Nr. 1, Februar 1999 (1999-02), Seiten 1-11, XP009024412 ISSN: 1087-2906
• DANIELS DION A ET AL: "A tenascin-C aptamer identified by tumor cell SELEX: Systematic evolution of ligands by exponential enrichment." PROCEEDINGS OF THE NATIONAL ACADEMY OF SCIENCES OF THE UNITED STATES OF AMERICA, Bd. 100, Nr. 26, 23. Dezember 2003 (2003-12-23), Seiten 15416-15421, XP002341306 ISSN: 0027-8424
• BLANK M ET AL: "Systematic evolution of a DNA aptamer binding to rat brain tumor microvessels: selective targeting of endothelial regulatory protein pigpen" JOURNAL OF BIOLOGICAL CHEMISTRY, AMERICAN SOCIETY OF BIOLOGICAL CHEMISTS, BALTIMORE, MD, US, Bd. 276, Nr. 19, 11. Mai 2001 (2001-05-11), Seiten 16464-16468, XP002978116 ISSN: 0021-9258
• PELSERS MAURICE M A L ET AL: "A sensitive immunoassay for rat fatty acid translocase (CD36) using phage antibodies selected on cell transfectants: Abundant presence of fatty acid translocase/CD36 in cardiac and red skeletal muscle and up-regulation in diabetes" BIOCHEMICAL JOURNAL, Bd. 337, Nr. 3, 1. Februar 1999 (1999-02-01), Seiten 407-414, XP002341307 ISSN: 0264-6021

EP 1 743 037 B1

**(Forts. nächste Seite)**

- FITZWATER AND B POLISKY T: "A SELEX primer" METHODS IN ENZYMOLOGY, ACADEMIC PRESS INC, SAN DIEGO, CA, US, Bd. 267, 1996, Seiten 275-301, XP002112701 ISSN: 0076-6879
- GOLD L ET AL: "DIVERSITY OF OLIGONUCLEOTIDE FUNCTIONS" ANNUAL REVIEW OF BIOCHEMISTRY, PALTO ALTO, CA, US, Bd. 64, Januar 1995 (1995-01), Seiten 763-797, XP000618016 ISSN: 0066-4154
- IRVINE D ET AL: "SELEXION. Systematic evolution of ligands by exponential enrichment with integrated optimization by non-linear analysis." JOURNAL OF MOLECULAR BIOLOGY. 5 DEC 1991, Bd. 222, Nr. 3, 5. Dezember 1991 (1991-12-05), Seiten 739-761, XP009052546 ISSN: 0022-2836
- HUMMEL V ET AL: "Production of MMPs in human cerebral endothelial cells and their role in shedding adhesion molecules" JOURNAL OF NEUROPATHOLOGY AND EXPERIMENTAL NEUROLOGY, Bd. 60, Nr. 4, April 2001 (2001-04), Seiten 320-327, XP009052425 ISSN: 0022-3069 in der Anmeldung erwähnt
- LYONS P D ET AL: "Cleavage of membrane-associated ICAM-1 from astrocytes: involvement of a metalloprotease." GLIA. FEB 1998, Bd. 22, Nr. 2, Februar 1998 (1998-02), Seiten 103-112, XP009052577 ISSN: 0894-1491
- DAVIES P ET AL: "Effects of cytochalasin B on endocytosis and exocytosis." FRONTIERS OF BIOLOGY. 1978, Bd. 46, 1978, Seiten 143-160, XP009052555 ISSN: 0071-965X
- GALBRAITH G M P ET AL: "METABOLIC AND CYTOSKELETAL MODULATION OF TRANSFERRIN RECEPTOR MOBILITY IN MITOGEN ACTIVATED HUMAN LYMPHOCYTES" CLINICAL AND EXPERIMENTAL IMMUNOLOGY, Bd. 42, Nr. 2, 1980, Seiten 285-293, XP009052587 ISSN: 0009-9104
- KAPLAN J ET AL: "TEMPERATURE SHIFTS INDUCE THE SELECTIVE LOSS OF ALVEOLAR MACROPHAGE PLASMA MEMBRANE COMPONENTS" JOURNAL OF CELL BIOLOGY, Bd. 94, Nr. 1, 1982, Seiten 12-19, XP009052580 ISSN: 0021-9525
- MÉTÉZEAU P ET AL: "Endocytosis of the membrane immunoglobulins of mouse spleen B-cells: a quantitative study of its rate, amount and sensitivity to physiological, physical and cross-linking agents." THE EMBO JOURNAL. OCT 1984, Bd. 3, Nr. 10, Oktober 1984 (1984-10), Seiten 2235-2242, XP009052582 ISSN: 0261-4189

**Beschreibung**

Die vorliegende Erfindung betrifft ein Verfahren zur Herstellung von spezifischen Molekülen, die hochselektiv an ausgewählte Zielmoleküle binden.

Für die Identifizierung biochemischer Moleküle wie z.B. Peptiden, Antikörpern, Nukleinsäuren oder chemisch-synthetischen Stoffen, die für diagnostische oder therapeutische Zwecke entwickelt werden sollen, stehen heute eine Reihe von Verfahren zur Verfügung. Die moderne Forschung mit dem Ziel der Wirkstofffindung hat sich dabei weitgehend auf die Verwendung von Molekül-Bibliotheken konzentriert. In diesen Bibliotheken werden die Biomoleküle zum Teil in großer Zahl, d.h. zwischen $10^9$ und $10^{15}$ verschiedener Spezies, zur Verfügung gestellt. Diese Bibliotheken können optimalerweise mittels unterschiedlicher "high throuput"-Verfahren durchsucht werden (Lenz, G.R. et al. (2000) Drug Discovery Today 5(4) 145-156; Jayasena, S. D. (1999). Clin. Chem. 45(9): 1628-1650; Khandurina, J. and A. Guttman (2002). Curr. Opin. Chem. Biol. 6(3), 359-366).

Solche relativ neuen Methoden werden schon im Vorfeld der Suche nach Wirkstoffen, z.B. zur funktionellen Charakterisierung neu entdeckter Proteine, einschließlich der Identifizierung ihrer Bindepartner, verwendet (MacBeath, G. and S. L. Schreiber (2000) Science 289(5485), 1760-1763). Vor allem werden Screening-Verfahren mit Hilfe von Molekülbibliotheken zum Auffinden von Wirkstoffen verwendet, die durch Bindung an Zielproteine (Zielmoleküle) identifiziert werden. Hierbei gibt es Bibliotheken für Makromoleküle, wie Peptide oder Nukleinsäuren (Aptamere) und kombinatorische Bibliotheken kleiner organischer Moleküle (Azriel-Rosenfeld, R. et al. (2004) J. Mol. Biol. 335(1): 177-192; Jayasena, S. D. (1999). Clin. Chem. 45(9): 1628-1650;; Khandurina, J. and A. Guttman (2002). Curr. Opin. Chem. Biol. 6(3), 359-366; MacBeath, G. and S. L. Schreiber (2000) Science 289(5485), 1760-1763).

Bei einer großen Zahl an Erkrankungen spielen Endothel- und Immunzellen eine zentrale Rolle und sind oft an der Entstehung ursächlich beteiligt. Dabei spielen sehr häufig auf der Oberfläche der Zellen vorhandene Membranmoleküle, deren Funktion durch spezifische Liganden aktiviert wird, eine ganz entscheidende Rolle. Nachfolgend sind stellvertretend hierfür nur einige wenige Beispiele genannt:

a) **Asthma:** Zellaktivierung und Entzündungsförderung durch bestimmte Interleukine wird bei Asthma beobachtet (Doucet, C., D. Brouty-Boye, et al. (1998) J. Clin. Invest. 101(10): 2129-2139 ; Larche, M., D. S. Robinson, et al. (2003) J. Allergy Clin. Immunol. 111(3): 450-463.

b) **Mukoviszidose** (Cyst. Fibrose): Chronische Entzündung der Atemwege wird durch stimulierende Faktoren verstärkt (Dudez, T. S., M. Chanson, et al. (2002) J. Infect. Dis. 186(6): 774-781).

c) **Fibrotic Lung Disease:** Anhaltende Entzündungsreahtionen führen zur Freisetzung von Wachstumsfaktoren, die erhöhte Zellteilung auslösen (Krein, P. M. and B. W. Winston (2002) Chest 122(6 Suppl): 289S-293S).

d) **Morbus Crohn und Ulcerative Colitis:** Bei beiden Darmerkrankungen fördern bestimmte Cytokine durch Bindung ihrer Rezeptoren auf der Zelloberfläche die entzündlichen Prozesse (Schmidt, C., T. Marth, et al. (2002) Pathobiology 70(3): 177-83; Murakami, H., S. M. Akbar, et al. (2002) Clin. Exp. Immunol. 128(3): 504-510.)

e) **Atherothrombosis bei Diabetes-Patienten**: Diabetes stellt ein Risiko für Atherothrombose und andere Gefäßerkrankungen dar, wobei Entzündungsmarker auf Immunzellen und Adhäsionsfaktoren eine ursächliche Rolle spielen (Berliner, S., O. Rogowski, et al. (2000) Cardiology 94(1): 19-25; Schram, M. T., N. Chaturvedi, et al. (2003) Diabetes Care 26(7), 2165-2173).

f) **Rheumatische Arthritis**: Die Erkrankung gilt als chronische Entzündung, bei der Immun-stimulierende und -inhibierende Faktoren aus dem Gleichgewicht geraten sind (Feldmann, M., et al. (1996) Cell 85(3): 307-310; den Broeder, A. A. et al. (2002) Ann. Rheum. Dis. 61(4): 311-318).

g) **Krebs**: Prozesse wie Zellwachstum, Angiogenese und Metastasierung von Tumoren benötigen die Interaktion von Membranrezeptoren der Tumorzelle mit spezifischen Liganden (Baselga J. (2001) Eur. J. Cancer 37 suppl 4: S16-S22; Ribatti, D., A. Vacca, et al. (2002) Eur. J. Cancer 38(6): 750-757 ; Pecheur, I., O. Peyruchaud, et al. (2002) Faseb J. 16(10): 1266-1268).

h) **Arteriosklerose**: Bei der Entstehung der Arteriosklerose spielen eine Vielzahl von Membranrezeptoren eine entscheidende Rolle (Freyberg, M. A., D. Kaiser, et al. (2001) Biochem. Biophys. Res. Commun. 286(1): 141-149; Lentsch, A. B. and P. A. Ward (2000) J. Pathol. 190(3): 343-348; Parker, C., 3rd, J. A. Vita, et al. (2001). Atheroslerosis 156(2): 417-424; Xu, Q. (2002) Arteriosler. Thromb. Vasc. Biol. 22(10): 1547-1559).

i) **Alzheimer**: Bei Alzheimer-Patienten kann das Amyloid-beta Protein eine Immunreaktion im Gehirn auslösen, die zum Krankheitsverlauf beiträgt (Giri, R., Y. Shen, et al. (2000) Am. J. Physiol. Cell Physiol. 279(6): C1772-81; Johnstone, M., A. J. Gearing, et al. (1999) J. Neuroimmunol. 93(1-2): 182-193).

Viele dieser Erkrankungen sind heute noch nicht oder nur sehr schwer heilbar. Dies liegt in einer Vielzahl der Fälle daran, dass es trotz der oben diskutierten Stoffbibliotheken, schneller und sensitiver Screening-Verfahren sowie von rekombinant herstellbaren Zielmolekülen in der Regel nicht gelang, Bindemoleküle zu isolieren, die unter *in vivo* Bedingungen eine hohe Wirksamkeit bzw. eine starke Bindung an das ausgewählte Zielmoleküle aufweisen.

Es besteht daher weiterhin auf dem Fachgebiet eine hohe Notwendigkeit, Wirkstoffe für medizinisch relevante Zielmoleküle zu entwickeln, wobei die medizinische Wirkung in den allermeisten Fällen durch Bindung des Wirkstoffs an das medizinisch relevante Ziehmolekül vermittelt wird.

Insbesondere hat es sich gezeigt, dass es mit den zur Zeit im Stand der Technik verwirklichten Screening-Verfahren nur selten und nur unter großen Schwierigkeit gelingt, gute Bindemoleküle für native Zellmembranmoleküle zu isolieren. Besonders das Einbeziehen der Zelloberfläche in die Wirkstoffentwicklung ist hier hervorzuheben, da mit Hilfe der bisherigen Verfahren wenn überhaupt überwiegend Binder gegen intrazelluläre Proteine oder extrazelluläre lösliche Proteine isoliert werden konnten.

Problematisch hieran ist unter anderem der nötige hohe Aufwand für die Isolierung der Zielmoleküle sowie die Tatsache, dass isolierte Membranproteine dann möglicherweise beim Screening-Verfahren in veränderter Struktur vorliegen. Diese würde dann nicht mehr der in vivo-Situation entsprechen, und somit würden die isolierten Bindemoleküle in vivo eine - wenn überhaupt vorhandene - gegenüber der experimentellen Situation verringerte Wirkung aufweisen.

Bisher im Stand der Technik veröffentliche Selektionsverfahren an Säugerzellen (Arap, W., M. G. Kolonin, et al. (2002) Nat. Med. 8(2): 121-127; Blank, M., T. Weinschenk, et al. (2001) J. Biol. Chem. 276(19): 16464-16468; Daniels, D. A., H. Chen, et al. (2003) Proc. Natl. Acad. Sci. U. S. A. 100(26): 15416-15421) wurden mit dem Ziel durchgeführt, zunächst ein breites Spektrum an bindenden Molekülen zu erhalten (Aptamere, Peptide), die möglichst einen Großteil der Oberflächenproteine der Zielzelle, z.B. einer Tumorzelle, als Targets abdecken sollten. Allenfalls hatte man hier noch die Option, im Laufe des Verfahrens oder anschließend eine Gegen-Selektion mit einer Kontroll-Zelle durchzuführen, um, wie im Fall der Tumorzelle als Zielobjekt, möglichst viele Bindemoleküle der Kontrollzelle (hier: Nicht-Tumorzelle) an dieser abzufangen und so zu eliminieren. Anschließend musste mit hohem Aufwand das zu jedem einzelnen erhaltenen Bindemolekül dazugehörige Target-Protein gesucht werden. Da heute viele Target-Proteine, die als medizinisch relevant gelten, bereits bekannt sind, sind diese Verfahren zur Wirkstofffindung gegen schon bekannte Proteine im allgemeinen viel zu aufwändig. Die Anwendbarkeit mit einer kompletten chemisch-synthetischen Bibliothek (Single Pool) ist bisher praktisch nur auf ein technisches Prinzip beschränkt: die spezifische Bindung an interessante Targets bleibt auf lösliche Proteine beschränkt, die erst nach Expression und Reinigung den synthetischen Molekülen als Bindepartner angeboten werden können.

Wirksame Substanzen können bislang an lebenden Zellen nur als einzelne Substanzen, nicht aber im Pool identifiziert werden, d.h. gerade hier sind Bindungstests bisher nur als rein analytische Tests in Form zahlreicher Einzeltests möglich. Gleiches gilt auch für funktionelle Tests an Zellen, wo z.B. die Modulierung zellulärer Punktionen, wie Zellteilung, oder die Aufnahme bestimmter Substanzen in die Zelle, mit jeder Einzelsubstanz mit großem Aufwand durchgeführt wird, d.h. nur in analytischer Form, nicht aber als präparatives Selektionsverfahren aus einem Pool von Substanzen. Wie bei anderen Verfahren zur Identifizierung von Substanzen aus Stoffbibliotheken (Antikörper-, Phage Display-, Peptid- und Nukleinsäure (Aptamer)-Bibliotheken) gibt es daher auch bei den als zukunftsträchtig geltenden synthetisch-chemischen Substanzbibliotheken bisher kein Verfahren, das auf die große Zahl der potenziellen Zielproteine auf Zelloberflächen, meist Rezeptoren in Zellmembranen, angewendet werden kann.

Daher besteht nach dem heutigen Stand der Technik ein großer Bedarf nach verbesserten Verfahren, mit deren Hilfe eine große Zahl von an der Entstehung von Krankheiten beteiligten Membranproteinen den modernen Methoden der Wirkstoffentwicklung zugänglich gemacht werden können.

Aufgabe der vorliegenden Erfindung war es daher, ein Verfahren zu entwickeln, mit dem spezifische Bindemoleküle gegen ausgewählte native Zielmoleküle hergestellt bzw. isoliert werden können. Insbesondere sollte es das Verfahren ermöglichen, Bindemoleküle gegen ausgewählte native Zielmoleküle hoch-selektiv, d.h. spezifisch nur auf ein einziges Zielmoleküle abzielend unter Ausschluss eines Maximums an unspezifisch bindenden Molekülen ohne Isolierung des Zielmoleküls isolieren zu können, einschließlich solcher Zielmolekül mit niedriger Kopienzahl und solcher, die z.B. nach Ligandenbindung durch Endozytose internalisiert werden.

Weitere Aufgaben der Erfindung ergeben sich aus der vorhergegangenen Diskussion des Stands der Technik.

Die Lösung des erfindungsgemäßen Problems ist in Anspruch 1 dargestellt. Bevorzugte Ausführungsbeispiele sind in den abhängigen Ansprüchen näher erläutert.

Dadurch, dass man

a) eine Stoffbibliothek enthaltend potentielle Bindemoleküle herstellt und mit den Zielmolekülen in Kontakt bringt,

b) einen unspezifischen Bindungskompetitor zugibt,

c) mindestens einmal ein Waschvorgang durchführt,

d) einen spezifischen Bindungskompetitor für das ausgewählte Zielmolekül in einer Konzentration, die mindestens etwa 10-fach, bevorzugt mindestens etwa 100-fach, besonders bevorzugt mindestens etwa 1000-fach höher ist als die Dissoziationskonstante $K_D$ (M) des Paares aus spezifischem Bindungskompetitor und Zielmolekül in der Stoffbibliothek zugibt und inkubiert, und

e) die nun abgelösten spezifischen Bindemoleküle mittels eines für die gewählte Stoffbibliothek geeigneten Verfahrens isoliert und/oder gegebenenfalls amplifiziert,

gelingt es auf erstaunlich einfache Art und Weise die oben genannte Aufgabe zu lösen.

Unter einem bevorzugten Gesichtspunkt ist die Stoffbibliothek eine amplifizierbare Stoffbibliothek ausgewählt aus der Gruppe bestehend aus: Nukleinsäure-Bibliotheken, Phage Display-Bibliotheken, Antikörper-Bibliotheken, Peptid-Bibliotheken oder eine synthetisch-chemische Bibliotheken, wie sie auf dem Fachgebiet gut bekannt und durch die im Fachgebiet publizierten Methoden leicht herstellbar sind. Gegebenenfalls kann eine solche Stoffbibliothek auch gekauft werden.

Da das erfindungsgemäße Verfahren prinzipiell sowohl für amplifizierbare als auch nicht-amplifizierbare Stoffbibliotheken durchführbar ist, können in Stufe a) verschiedene Bibliotheken eingesetzt werden. Diese sind entweder kommerziell erhältlich oder können vor Durchführung des Selektionsverfahrens mit Hilfe von im Stand der Technik gut bekannten Methoden einfach hergestellt werden.

Unter einem weiteren bevorzugten Gesichtspunkt werden die Stufen a) und b) des oben angegebenen Verfahrens zeitgleich durchgeführt.

Unter einem weiteren bevorzugten Gesichtspunkt wird der unspezifische Kompetitor ausgewählt aus der Gruppe bestehend aus: Milchpulver, Serum-Proteine wie Serum-Albumin oder Nukleinsäuren wie tRNA oder Hefe-RNA, Erythrozyten, Erythrozyten-Ghosts (Membran-Hüllen), Zelllinien hematopoetischen Ursprungs, wie Lymphoma- oder Leukämie-Zellen, oder HeLa-Suspensionszellen, sowie Zellorganellen wie Zellkerne oder Mitochondrien, wobei die Zellen, Zellmembranen oder Organellen das Zielmolekül nicht tragen, bevorzugt sind dabei Erythrozyten, Erythrozyten-Ghosts (Membran-Hüllen), Zelllinien hematopoetischen Ursprungs, wie Lymphoma- oder Leukämie-Zellen, oder HeLa-Suspensionszellen, sowie Zellorganellen wie Zellkerne, Mitochondrien, wobei die Zellen, Zellmembranen oder Organellen das Zielmolekül nicht tragen.

Ganz besonders bevorzugt handelt es sich bei dem unspezifischen Kompetitor um Erythrozyten und/oder Erythrozyten-Ghosts (Membran-Hüllen), die das Zielmolekül nicht tragen.

Unter einem weiteren bevorzugten Gesichtspunkt werden die Stufen a) bis e) mehrmals, bevorzugt jedoch nicht mehr als drei bis fünfmal aufeinander folgend, durchgeführt, wobei ab der zweiten Runde die in Stufe e) erhaltenen Bindemoleküle in Stufe a) anstelle der amplifizierbaren Bibliothek eingesetzt werden.

Unter einem weiteren bevorzugten Gesichtspunkt handelt es sich bei der Bibliothek um eine Aptamer-Bibliothek. Methoden zur Herstellung solcher Aptamer-Bibliotheken sind gut bekannt, und gehen im allgemeinen auf die US 5,475,096 zurück, worin entsprechende Methoden offenbart sind. Diese Methoden wurden weiter entwickelt, und stehen dem Fachmann in einer überwältigenden Vielzahl von Varianten zur Verfügung.

Unter einem weiteren bevorzugten Gesichtspunkt liegen die Zielmoleküle in Stufe a) des erfindungsgemäßen Verfahrens nativ, insbesondere im Verbund mit einer Biomembran, bevorzugt auf der Oberfläche einer Zellmembran, insbesondere bevorzugt auf intakten Zellen vor.

Unter einem weiteren bevorzugten Gesichtspunkt wird in Stufe a) ein Endozytose-Inhibitor in ausreichender Menge hinzugegeben, um die Endozytose zu inhibieren, bevorzugt Cytochalasin B in einer Menge von 1 bis 10 $\mu$M.

Unter einem weiteren bevorzugten Gesichtspunkt ist das ausgewählte Zielmoleküle ein membranständiges Molekül, von dem auch eine lösliche Form bekannt ist, und es wird in Stufe a) ein Proteinase-Inhibitor zugegeben.

Unter einem weiteren bevorzugten Gesichtspunkt ist der spezifische Bindungskompetitor ausgewählt aus der Gruppe bestehend aus Antikörpern und deren Fragmenten, Aptameren, Liganden der Zielmolekül sowie niedermolekularen Kompetitoren.

Insbesondere bevorzugt handelt es sich dabei um Liganden für klinisch oder diagnostisch oder sonst wie interessante Oberflächenmoleküle von Zellen, besonders bevorzugt IAP oder $\alpha v \beta 3$.

Insbesondere ist es von Vorteil, dass mit dem erfindungsgemäßen Verfahren gerade solche Bindemoleküle hergestellt werden können, die spezifisch die Wechselwirkung zwischen Ligand und Zielmolekül stören, und damit mit gegenüber den im Stand der Technik bekannten Verfahren stark erhöhter Wahrscheinlichkeit funktionelle Binder, d.h. solche, die die Wechselwirkung des z.B. natürlichen Liganden mit dem Zielmolekül imitieren oder inhibieren. Es gelingt auf diese Weise erstaunlich schnell und einfach, biologisch aktive Bindemoleküle, und somit potentiell wirksame Wirkstoffe, herzustellen.

Unter einem weiteren bevorzugten Gesichtspunkt betrifft die vorliegende Erfindung einen Kit, umfassend mindestens einen unspezifischen Bindungskompetitor und einen Endozytose-Inhibitor und/oder einen Proteaseinhibitor.

Der Kit kann außerdem eine amplifizierbare Stoffbibliothek umfassen.

Weiterhin betrifft die vorliegende Erfindung auch die Bindemoleküle, die mithilfe des erfindungsgemäßen Verfahrens isoliert wurden sowie deren Verwendung, z.B. in Therapie oder Diagnose.

Unter einer amplifizierbaren Stoffbibliothek wird für die Zwecke der vorliegenden Erfindung mit Vorteil eine Bibliothek ausgewählt aus der Gruppe bestehend aus Nukleinsäure-Bibliotheken, Phage Display-Bibliotheken, Antikörper-Bibliotheken oder chemisch-synthetischen Bibliotheken verstanden.

Unter "spezifische Bindemoleküle" werden daher erfindungsgemäß solche Moleküle verstanden, wie sie Gegenstand der zahlreichen im Stand der Technik bereits verwirklichten Stoffbibliotheken sind. Hierzu gehören beispielsweise Aptamere, Antikörper und deren Fragmente, Peptide, Anticaline und chemisch synthetisierte Moleküle.

Aus der Art der gewählten und hergestellten Stoff-Bibliothek ergibt sich dabei auch zwanglos das daraus zu isolierende potentielle Bindemolekül.

Der spezifische Bindungskompetitor, der Affinität zum Zielprotein besitzt, wird mit Vorteil ausgewählt aus der Gruppe bestehend aus Antikörpern, Antikörper-Fragmenten, Peptiden, DNA- und RNA-Aptameren, niedermolekularen Verbindungen und anderen, z.B. natürlich vorkommenden Liganden des Zielproteins.

Dieser spezifische Bindungskompetitor wird abhängig von seiner Affinität zum Zielmolekül in einer Konzentration von mindestens 1 $\mu$M eingesetzt, bevorzugt von mindestens 10$\mu$M, besonders bevorzugt von mindestens 100$\mu$M, ganz besonders von mindestens 1mM und am meisten bevorzugt von mindestens 10 mM. Es ist jedoch selbstverständlich, dass diese Konzentration aufgrund der Verfügbarkeit und /oder der Löslichkeit des spezifischen Bindungskompetitors eine Obergrenze haben muss, die vom Fachmann jeweils mit großer Leichtigkeit ermittelt werden kann.

Dabei wird der Fachmann im Rahmen der experimentellen Möglichkeiten eine möglichst hohe Konzentration an spezifischem Bindungskompetitor bevorzugen. Naturgemäß ist über die Affinität des potentiellen Bindemoleküls aus der Stoffbibliothek vor Durchführung des Experiments nichts bekannt. Im Allgemeinen sollen bevorzugt starke Binder isoliert werden, so dass eine möglichst hohe Konzentration an spezifischem Bindungskompetitor wünschenswert ist.

Ist die Bindungskonstante des spezifischen Kompetitors für das Zielmolekül bekannt oder zumindest abschätzbar, so liegt die Konzentration des Bindungskompetitors mindestens etwa 10-fach, bevorzugt mindestens etwa 100-fach, besonders bevorzugt mindestens etwa 1000-fach höher als der Wert der bekannten oder ermittelten oder abgeschätzten Bindungskonstanten ($K_D$).

Bekannte Werte für Bindungskonstanten ($K\{D\}$) der Bindung von Antikörpern bzw. Antikörper-Fragmenten an Proteine sind z.B. $10^{-10}$, bis $10^{-8}$ M (Hornick, J. L., J. Sharifi, et al. (2000) J. Nucl. Med. 41(2): 355-362; Nielsen, L. S., J. G. Hansen, et al. (1983) Embo J. 2(1): 115-119; Zhou, Y. H., M. Takekoshi, et al. (2002) Antiviral Res. 56(1): 51-59), für Aptamere $10^{-9}$ bis $10^{-7}$ M (Rhodes, A., A. Deakin, et al. (2000) J. Biol. Chem. 275(37): 28555-28561; Martell, R. E., J. R. Nevins, et al. (2002) Mol. Ther. 6(1): 30-34) bzw. für Peptide $10^{-5}$ bis $10^{-8}$ M (Lamla, T. and V. A. Erdmann (2003) J. Mol. Biol. 329(2): 381-388; Nord, K., O. Nord, et al. (2001) Eur. J. Biochem. 268(15): 4269-4277).

Unter einem besonders bevorzugten Ausführungsbeispiel betrifft vorliegende Erfindung weiterhin einen Kit of Parts, umfassend mindestens einen unspezifischen Bindungskompetitor sowie einen spezifischen Bindungskompetitor, besonders bevorzugt weiterhin umfassend eine amplifizierbare Stoffbibliothek.

Mit dem erfindungsgemäßen Selektionsverfahren können prinzipiell gegen alle biologischen Zielnoleküle auf Zelloberflächen therapeutisch oder diagnostisch funktionale Moleküle selektiert werden, vorausgesetzt, es steht ein spezifischer Ligand, Antikörper oder anderes Bindemolekül für das Zielmoleküle bereits zur Verfügung.

Überraschenderweise hat sich gezeigt, dass mit dem erfindungsgemäßen Verfahren bereits nach etwa 5 Selektionsrunden effektive Binder gefunden wurden. Im Stand der Technik wurden bei anderen Verfahren, nämlich den sogenannten SELEX-Verfahren zur Identifizierung von bindenden Nukleinsäuren aus einem Pool von ca. $10^{14}$ und mehr Molekülen bisher eine wesentlich höhere Anzahl von Selektionsrunden benötigt. Erfindungsgemäß wird die Selektion nämlich nicht so lange fortgeführt, bis, wie in SELEX-Verfahren, Molek-ülfamilien gefunden wurden, was in der Regel mindestens acht bis zehn Selektionsrunden benötigt. Dagegen wird erfindungsgemäß nach Erreichen einer apparent höheren Affinität im Selektionspool, was überraschenderweise bereits nach etwa fünf Selektionsrunden der Fall ist, die Affinität einzelner Moleküle an das Zielmolekül mit dem gleichen Verfahren wie in der präparativen Selektion bestimmt. Durch diese Vorgehensweise wird ausgeschlossen, dass nach zu vielen Selek-tionsrunden eine kleine Gruppe von Zielmoleküle (bzw. daran bindender Moleküle) herausselektiert wird, die nicht mehr das gesamte oder einen Großteil des Spektrums an potentiellen Zielmolekülen repräsentiert. Im Unterschied zu den SELEX-Verfahren bei Nukleinsäuren ist diese Vorgehensweise (ohne extensive Suche nach Molekülfamilien) wohl auch deshalb von Bedeutung, da sich viele Zielmoleküle in einem erheblichen molekularen Unterschuss von mehreren Zehnerpotenzen gegenüber allen anderen Zielmoleküle befinden: Ein Membranprotein mit einer Kopienzahl von 10.000 pro Zelle wird nicht nur gegen einige Hundert oder wenige Tausend andere Molekülspezies der jeweiligen Zelloberfläche selektiert, sondern mit diesem Zielmolekül muss auch noch in Konkurrenz zu vielen anderen Zielmolekülen mit einer höheren Kopienzahl (von einigen Zehntausend bis wenigen Hunderttausend pro Zelle) effizient selektiert werden können; daraus ergibt sich im ungünstigsten Fall ein molekularer Überschuss von etwa $10^4$ bis $10^5$ der unerwünschten Moleküle gegen das (als Zielmolekül) erwünschte Molekül.

Die Selektion wird erfindungsgemäß im Falle von Proteinen als Zielmolekül bevorzugt nicht mit isoliertem Ziel-Protein, sondern mit intakten Zellen oder Zellmembranen durchgeführt, wobei erfindungsgemäß eine intakte Zellmembran auch eine solche einschließt, die nach Aufbrechen der Zellen, z.B. durch aufeinanderfolgendes Einfrieren und Auftauen der Zellen, präpariert wird. Durch dieses Vorgehen wird keine strukturelle Veränderung durch künstliche Expression, z.B. in Bakterien, und Reinigung des Target-Proteins in Kauf genommen. Weiterhin wird dadurch erreicht, dass sich das Protein noch im Komplex mit anderen Proteinen befindet.

Wird an lebenden Zellen bzw. auf ihrer Oberfläche eine Selektion mit Molekülbibliotheken vorgenommen, kann bei Temperaturen oberhalb von 4°C eine Endozytose des jeweiligen Membranproteins, das als Zielmolekül gerade gebunden wird, nicht ausgeschlossen werden. Die Endozytose kann auch durch die Bindung des jeweiligen Moleküls bzw. Bakteriophagen ausgelöst werden, wenn dabei die Interaktion mit einem Liganden imitiert wird. Auch wenn der Prozentsatz der Proteine, die internalisiert werden, gering ist, wird er sich doch über den gesamten Zeitraum der jeweiligen Selektion summieren, so dass z.B. etwa drei Stunden nach der initialen Bindung der Bindepartner aus Molekülbibliothek und nachfolgendem Waschen bzw. Eluieren nur noch ein sehr kleiner Teil von wenigen Prozent übrig bleibt, d.h. als Komplex an der Zelloberfläche vorliegt. Dies ist deshalb wichtig, weil, im Unterschied zu anderen Verfahren, die noch gebundenen Moleküle bzw. Partikel nicht durch Zellaufschluss und anschließende Isolierung, sondern durch Freisetzen von der Oberfläche zurückgewonnen werden. Um dieses Problem zu umgehen, wird im Falle der Verwendung von lebenden Zellen ein Inhibitor der Endozytose zugesetzt, z.B. Cytochalasin B.

An Zelloberflächen gebundene Liganden können auch durch proteolytische Freisetzung (Hummel, V., B. A. Kallmann, et al. (2001) J. Neuropathol. Exp. Neurol. 60(4): 320-327.) der Proteine, an die sie gebunden sind, verloren gehen: So gibt es zusätzlich zu den membranständigen Proteinen der Selektin- Familie oder der "cell adhesion molecule" (CAM)-Familie (ICAM, VCAM) die entsprechenden löslichen Proteine (sICAM, cVCAM), die z.B. auch im Blut nachweisbar sind (Mukae, H., J. Ashitani, et al. (2003) Respirology 8(3): 326-331). Um Verlust der gebundenen Moleküle zu vermeiden, können bei Selektion gegen Membranproteine dieser Klasse entsprechende Protease-Inhibitoren den entsprechenden Bindungs- und Wasch-Lösungen zugesetzt werden.

Erfindungsgemäß wird damit ein Verfahren etabliert, bei dem i) Oberflächenmoleküle auf Säugerzellen, Zellmembranen und Membranen, ii) Oberflächen von Organellen, oder Zellmembranen, iii) Zellen, in denen das Zielmolekül, eventuell zusammen mit anderen Zielmolekülen, als Membranmolekül exprimiert wurde, iv) und Moleküle als Bestandteil einer extrazellulären Matrix, als Zielmoleküle für eine Wirkstoff-Entwicklung mit Hilfe von Molekül-Bibliotheken zugänglich macht. Dies geschieht durch Bindung an die Zelle, Organelle, Membran oder extrazelluläre Matrix in Gegenwart bestimmter Zusätze, Abwaschen nicht gebundener oder schwach gebundener Moleküle bzw. Partikel und Ablösen der noch gebundenen durch Kompetition mit einem bekannten Liganden, z.B. einem Antikörper, im Überschuss. Anschließend kann entweder (a) direkt die Analyse bzw. Identifizierung der gebundenen Moleküle erfolgen; oder (b) die Analyse und Charakterisierung folgt auf Schritte zur Isolierung und Amplifizierung (z.B. durch Klonieren) der gebundenen Moleküle; oder (c) das Verfahren wird noch einmal oder mehrere Male wiederholt, wobei das Gemisch der gebundenen Moleküle direkt oder nach Amplifikation für die Bindung eingesetzt wird. In diesem Fall (Fall "c") wird nach der Amplifikation (in vitro oder in vivo) die gebundene Menge der letzten Selektion bestimmt, daraus die relative Affinität der jeweiligen Selektionsrunde abgeschätzt und der gesamte Selektionsvorgang wiederholt. Wird eine erhöhte Bindung nach mindestens drei bis fünf Runden beobachtet, werden einzelne Bindemoleküle isoliert und deren relative Affinität bestimmt. Dies kann nach dem gleichen Verfahren wie im eigentlichen Selektionsverfahren geschehen, d.h. durch Bindung und anschließende Kompetition durch den gleichen Antikörper bzw. Liganden sowie quantitative Bestimmung der gebundenen Menge des jeweiligen Moleküls; als alternative Methode hierzu kann z.B. die Bindung des Liganden bzw. Antikörpers, der im Selektionsverfahren benutzt wurde, verfolgt werden, wobei durch Zugabe einer definierten Konzentration des jeweiligen Bindemoleküls als Kompetitor dessen Bindung verhindert wird und aktive Moleküle identifiziert werden.

Das abgelöste und in Lösung oder in Suspension befindliche spezifische Bindemolekül wird also entweder durch entsprechende Analyse direkt identifiziert, oder mittels eines für die gewählte amplifizierbare Stoffbibliothek geeigneten Amplifikationsverfahrens vermehrt und anschließend mittels geeigneter Verfahren isoliert und identifiziert.

Als unspezifischer Bindungskompetitor können eine Vielzahl von Stoffen eingesetzt werden wie beispielsweise Milchpulver, Serum-Proteine wie Serum-Albumin oder Nukleinsäuren wie tRNA oder Hefe-RNA. Bei der Verwendung von lebenden Zellen als Membran hat es sich als vorteilhaft erwiesen, in dem erfindungsgemäßen Verfahren als unspezifischen Bindungskompetitor zusätzlich Erythrozyten einzusetzen. Alternativ können auch Erythrozyten-Ghosts (Membran-Hüllen), oder verschiedene andere Zellen bzw. ihre Membranen als Bindungskompetitoren eingesetzt werden, wie z.B. Zelllinien hematopoetischen Ursprungs, wie Lymphoma- oder Leukämie-Zellen, oder HeLa-Suspensionszellen, sowie Zellorganellen wie Zellkerne, Mitochondrien und andere. Voraussetzung hierfür ist, dass die Zellen, Zellmembranen oder Organellen das Zielprotein (Target) des jeweiligen Verfahrens nicht in ihrer Membran tragen.

Erfahrungsgemäß stellen bei solchen Selektionsverfahren meistens weniger konkurrierende Zielmoleküle ein Problem dar, als vielmehr die unspezifische Bindung der verwendeten Stoffklasse der potentiellen Bindemoleküle (z.B. Nukleinsäuren, Proteine). Beide können eine Vielzahl von Interaktionen mit prinzipiell allen biologischen Molekülen eingehen, bei einer Zelloberfläche stellt sich vor allem das Problem der großen Anzahl an Wasserstoffbrücken-Bindungen, die mit Proteinen, aber besonders mit glykosylierten Proteinen bzw. Lipiden, eingegangen werden können. Ob diese als "spezifisch" oder "unspezifisch" bezeichnet werden, ist in den meisten Fällen nicht von Bedeutung; aber bei der schwierigen Aufgabe, an seltenen Proteinen auf Zelloberflächen als Zielmoleküle zu selektieren, kann eine auch nur schwache Bindung an eine Überzahl von Zuckermolekülen die Aufgabe zu stark erschweren. Daher werden erfindungsgemäß zur Vermeidung einer unspezifischen Zellbindung bevorzugt andere Zellen, hier Erythrozyten, als erfindungsgemäße unspezifische Kompetitoren in die Bindungs-Lösung und in bestimmte Waschlösungen zugegeben. Dies hat auch den praktischen Vorteil, dass die Effizienz der Waschvorgänge, wo keine Erythrozyten zugesetzt waren, optisch durch die nachlassende Rotfärbung gut verfolgt und gegebenenfalls weiter gewaschen werden kann. Das Verfahren erlaubte eine Selektion von hochspezifisch bindenden RNA-Molekülen aus einer Bibliothek nach nur fünf Runden.

Unter ausgewählten Zielmolekülen werden erfindungsgemäß bevorzugt Moleküle verstanden, die *in vivo* in bzw. auf der Membran von Zellen, bevorzugt Säugerzellen vorliegen, und für die ein hochspezifischer Ligand wie z.B. ein Antikörper, ein Aptamer oder ein anderer Ligand bereits existiert. Bei diesen Zielmoleküle handelt es sich im Allgemeinen um Proteine oder Zuckermoleküle. Es können jedoch auch Komplexe aus beiden Stoffklassen oder Nukleinsäuren bzw. Derivate und andere, auf bzw. in biologischen Membranen vorkommende Moleküle sein.

Selbstverständlich kann dieser Ligand auch erst direkt vor Durchführung des Verfahrens isoliert worden sein.

Die Selektion erfolgt erfindungsgemäß in einem wässrigen Medium. Darunter werden Puffersysteme verstanden, wie sie auf dem Fachgebiet gut bekannt sind. Insbesondere handelt es sich um Phosphat-Puffer, HEPES-Puffer und Tris-Puffer.

Zwischen Stufe b) und d) des in Anspruch 1 definierten erfindungsgemäßen Verfahrens wird ein Waschvorgang durchgeführt. Dieser dient dazu, ungebundenes Material zu entfernen, und bedient sich normalerweise des gleichen Mediums wie Stufe a) und b), mit oder ohne unspezifischen Bindungskompetitor.

Als spezifischer Bindungskompetitor wird der oben schon erwähnte spezifische Ligand eingesetzt. Aus Stufe e) wird das (die) selektionierte(n) Bindemoleküle anschließende mittels geeigneter Verfahren amplifiziert bzw. isoliert und charakterisiert. Diese Verfahren sind abhängig von der Art der gewählten Stoffbibliothek, und dem Fachmann jeweils klar und geläufig. Beispielsweise werden bei Aptamer-Bibliotheken die Aptamere mit Hilfe einer PCR-Reaktion amplifiziert. Bedient man sich der Phage Display oder verwandter Technologien, so werden die selektionierten Phagenpartikel in Bakterienzellen, meist *E. coli,* vermehrt.

Bei synthetisch-chemischen (kombinatorischen) Bibliotheken werden die selektierten Moleküle mit geeigneten Verfahren identifiziert. Beispielsweise sind im Stand der Technik Codierungsverfahren bekannt, die über die kovalente Ankopplung der chemisch-synthetischen Moleküle an (i) Träger-Partikel in Form einer chemischen Codierung (Blackwell, H. E., L. Perez, et al. (2001) Chem. Biol. 8(12): 1167-1182; Clemons, P. A., A. N. Koehler, et al. (2001) Chem. Biol. 8(12): 1183-1195) bzw. an (ii) Bakteriophagen als biochemische (genetische) Codierung (Woiwode, T. F., J. E. Haggerty, et al. (2003) Chem. Biol. 10(9): 847-858) eine spätere Identifizierung der gebundenen Moleküle aus der Molekülbibliothek ermöglicht. Der Vorteil des zweiten Verfahrens besteht darin, dass durch die Kopplung an codierte Bakteriophagen die gesamte durch kombinatorische Synthese erhaltene Molekül-Bibliothek als "Single Pool", also als Mischung aus mehreren Hundert (hier: 980) chemischen Substanzen in einem einzigen Schritt eingesetzt werden kann. Daher entfällt eine äußerst aufwändige Logistik, die bei Bindungstests mit allen Substanzen einzeln nötig wäre (Fellouse, F. & K. Deshayes (2003) Chem. Biol. 10(9): 783-786).

Der Ausdruck "nativ" bzw. "natives Protein, Molekül" wird vorliegend verwendet, um solche Moleküle zu kennzeichnen, die in einer Konformation und Umgebung vorliegen, wie es der in vivo Situation entspricht, so dass volle Funktionalität sowohl des Moleküls als auch der aufgefundenen Bindemoleküle gewährleistet ist.

Insbesondere handelt es sich dabei um Moleküle auf der Oberfläche von Zellen. Mitumfasst sind dabei auch rekombinante Moleküle, die von Zellen exprimiert werden, und sich z.B. auf der Zelloberfläche, der Oberfläche von Zellorganellen oder künstlichen Vesikeln und anderen Konstrukten, die die Membranumgebung imitieren, befinden.

Erfindungsgemäß geeignete Endozytose-Inhibitoren umfassen beispielsweise Cytochalasin B, Cytochalasin D, Cytochalasin E, Latrunculin A und -B, Swinholide A und andere Stoffe, die die Funktion des Cytoskeletts blockieren. Erfindungsgemäß wird unter Kit eine Aufmachung verstanden, in denen mindestens ein spezifischer und ein unspezifischer Bindungskompetitor, gegebenenfalls zusammen mit einer amplifizierbaren Stoffbibliothek vertrieben wird.

Die nachfolgenden Beispiele erläutern die Erfindung ohne diese auf die gezeigten Ausführungsbeispiele zu beschränken. Stellvertretend für andere Selektions-tionssysteme werden hier mit Hilfe des erfindungsgemäßen Verfahrens Aptamere selektiert, die spezifisch an IAP binden, und dadurch die durch TSP-1 induzierte Apoptose von kultivierten Endothelzellen inhibieren (Freyberg et al., BBRC 2000, 271, Nr. 3, Seiten 584-588).

Die folgende Übersicht zeigt die verwendeten Materialien und Bezugsquellen:

| Material | Firma, Ort | Best.-Nr | Bemerkung |
|---|---|---|---|
| Phenol | Carl Roth, Karlsruhe | 0038.1 | TE-äquilibriert |
| Taq Polymerase | Peqlab, Erlangen | 01.1030 | |
| T7-RNA Polymerase | MBI-Fermentas, St. Leon-Roth | EP0111 | |
| RevertAid M-MuLV-Reverse Transcriptase | MBI-Fermentas, St. Leon-Roth | EP0441 | |
| ATP, GTP | Carl Roth, Karlsruhe | K045.1 K047.1 | |
| 2'-NH$_2$-UTP 2'-NH$_2$-CTP | tebu-bio, Offenbach | N-1027 N-1026 | Hersteller: TriLink, San Diego |
| RQ1DNase I, RNase frei | Promega, Mannheim | M6101 | |
| Chemikalien, | Carl Roth, Karlsruhe | | |
| Biochemikalien | Merck, Darmstadt; Sigma, Deisenhofen | | |

synthetische DNA:

primer A: GCGAAGCTTTAATACGACTCACTATAGGGAGACGATATTCGTCCATC

primer B: GGTCGAGAATTCAGTCGGACAGCG

Lib40: GAATTCAGTCGGACAGCG(N$_{40}$)GATGGACGAATATCGTCTCCC

Bidestilliertes Wasser zum Ansetzen von Lösungen wurde mit Diethylpyrocarbonat (DEPC), 0,01 % (v/v) für 24 Stunden bei 20 bis 25°C behandelt und anschließend für 60 min bei 121°C autoklaviert; es wird dann mit "DEPC-H$_2$O" bezeichnet.

**Lösungen:**

| | |
|---|---|
| TE-Puffer | 10 mM Tris-HCl, 0,1 mM EDTA, pH 7,6 |
| CI | Chloroform/Isoamylalkohol-Mischung 24:1 (Volumen/Volumen) |
| 1xTBE | 90 mM Tris, 90 mM Borsäure, 1 mM EDTA |
| 1xPBS+Mg | 130 mM NaCl, 15,4 mM Na$_2$HPO$_4$ , 4,6 mM NaH$_2$PO$_4$, 5 mM MgCl$_2$ |
| Wp3E | 65 mM NaCl, 7,7 mM Na$_2$HPO$_4$, 2,3 mM NaH$_2$PO$_4$, 2,5 mM MgCl$_2$, 0,25 mM CaCl$_2$, 12 μg/ml tRNA (Hefe), 50 % (v/v) Erythrozyten-Suspension |
| Wp3EC | wie Wp3E + Cytochalasin B (Endkonzentration 5 μM) |
| WP3 | 130 mM NaCl, 15,4 mM Na$_2$HPO$_4$ 4,6 mM NaH$_2$PO$_4$ , 5 mM MgCl$_2$, 0,5 mM CaCl$_2$, 12 μg/ml tRNA (Hefe) |
| Wp3T | 130 mM NaCl, 15,4 mM Na$_2$HPO$_4$ 4,6 mM NaH$_2$PO$_4$ , 5 mM MgCl$_2$, 0,5 mM CaCl$_2$, 12 μg/ml tRNA (Hefe), Tween 20 (0,1 % {v/v}) |

**Beispiel 1**

Konstruktion einer Nukleotidsequenz-Bank als PCR-DNA

Zur Konstruktion einer Aptamer-Bank wurde eine DNA durch PCR synthetisiert nach den Vorgaben, wie sie von M. Homann und H.U. Göringer {(1999) Nucleic Acids Res., 27 (9), 2006-2014} beschrieben sind, mit den drei beschriebenen Oligonukleotiden d.h. ein 79 Nukleotide langes DNA-Oligonukleotid als Matrize, genannt "Lib40", das einen 40 Nukleotide langen Bereich mit einer zufälligen Sequenz enthielt, genannt "N40", wobei N für jede der vier Nukleotide, A, G, C oder T steht, und zwei Oligonukleotide, die als Primer bei der PCR fungierten, genannt "primer A" und "primer B"; bei der PCR entsteht eine doppelsträngige DNA, bei der durch den Promotor der T7 RNA Polymerase die "N40"-Sequenz in RNA abgeschrieben werden kann. Es wurden 20 PCR-Ansätze parallel durchgeführt, jeder PCR-Ansatz enthielt in einem Volumen von 200 $\mu$l: 160 pmol "primer A", 160 pmol "primer B", 74,2 pmol Lib40-DNA, Pufferlösung wie vom Hersteller des Enzyms Taq-Polymerase (Peqlab, Puffer "blau") vorgeschrieben, hier mit einer End-konzentration MgCl$_2$ von 1,5 mM, vier Nukleosidtriphosphate dATP, dGTP, dCTP und dTTP mit je 0,25 mM, und das Enzym Taq-Polymerase, 7,5 units. PCR-Protokoll: 20 sec 94°C, dann PCR-Zyklus: 94°C für 70 sec, 44°C für 90 sec, 72°C für 70 sec, durchgeführt wurden hier 8 Zyklen, dann wurde auf 4°C abgekühlt. Die Reinigung erfolgte durch Phenolextraktion, dann Extraktion mit CI (Chloroform/Isoamylalkohol, 24:1 Volumenverhältnis [v/v]), und eine Ethanolfällung durch Zugabe von Natriumacetat, pH 5,6, auf eine Konzentration von 0,3 M, und dem 2,5-fachen Volumen Ethanol p.a., dann wurde mindestens 30 min bei - 20°C inkubiert und dann 25 - 35 min bei 20.000 - 21.000 g und 4-18 °C zentrifugiert. Nach Entfernen des Überstands wird der Niederschlag mit 70 % (v/v) Ethanol p.a. gewaschen. Der Niederschlag wird in insgesamt 100 $\mu$l TE-Puffer gelöst und bei - 20°C aufbewahrt.

**Beispiel 2**

Synthese der Y-Amino-RNA

Ausgehend von doppelsträngigen PCR-DNAs (jeweilige PCR-Ansätze: für Selektionsrunde 1: 2,0 bis 2,3 nmol DNA aus Beispiel 1; nachfolgende Selektionsrunden: 1,0 bis 1,3 nmol DNA, aus Beispiel 5, PCR "P2"; analytischer Ansatz: 60 bis 70 pmol DNA aus zweiter PCR, Beispiel 6), die die Promotor-Sequenz des T7 Bakteriophagen enthalten (siehe Beispiel 1), wird die Y-Amino-RNA durch die T7 RNA Polymerase synthetisiert. Mit Y-Amino-RNA ist eine modifizierte RNA gemeint, bei der die Pyrimidine (Nukleotide U und C) an der 2'-Position statt der OH-Gruppe eine NH$_2$-(Amino-) Gruppe tragen. Die Transkription in Gegenwart von ATP und GTP (je 1 mM) sowie der modifizierten Nukleotide 2'-NH$_2$-UTP und 2'- NH$_2$-CTP (je 1 mM; Modifizierung siehe oben, Ersetzen der Hydroxylgruppe durch eine Aminogruppe in 2'-Position) tragen, durch Inkubation mit der käuflichen T7 RNA Polymerase (2 units/$\mu$l) in Gegenwart der vom Hersteller empfohlenen Bedingungen und zusätzlich 2 mM MgCl$_2$, in einem Volumen von 400 $\mu$l (Selektionsrunde 1: 800 $\mu$l; analytischer Ansatz: 40 $\mu$l) bei 16°C +/- 3 °C für 20 bis 44 Stunden inkubiert. Dann wird die noch vorhandene DNA durch Zusatz von MgCl$_2$ (Erhöhung der Konzentration um 2,74 mM) und RNase-freie DNase I (Endkonzentration 0,06 units/ $\mu$l) und Inkubation bei 37°C für 135 min verdaut, wobei 1 unit DNase I definiert ist als die Enzymmenge, die 1 $\mu$g DNA in 50 $\mu$l in Puffer mit 40 mM Tris-HCl, 10 mM NaCl, 6 mM MgCl$_2$, 10 mM CaCl$_2$, (pH 7,9 bei 25°C), in 10 Minuten bei 37°C komplett verdaut.

Nach Zugabe des gleichen Volumens TE-Puffer wird die Lösung zunächst mit Phenol und dann mit Chloroform/ Isoamylalkohol (24:1- {v/v}) extrahiert. Danach wird die Y-Amino-RNA mit Ethanol gefällt (siehe Beispiel 1) und nach Waschen mit 70 % Ethanol in 300 $\mu$l (Selektion 1: 1,2 ml; analytischer Ansatz: 30 $\mu$l) DEPC-H$_2$O gelöst. Die Ausbeute an Y-Amino-RNA wird durch Analyse nach Trennung in einem Polyäcrylamid-Gel (9,47 % (w/v) Acrylamid und 0,53 % Bis-Acrylamid, 8 M Harnstoff und 1xTBE-Puffer) in Gegenwart von 1xTBE-Puffer bestimmt, die Trennung der Nukleinsäuren erfolgte bei 12 bis 16 V/cm für 35 bis 45 min. Das Gel wurde angefärbt mit dem Fluoreszenzfarbstoff SybrGreen II (Molecular Probes; Konzentration nach Herstellerangabe) für 20 min und unter UV-Licht die Fluoreszenz im sichtbaren Bereich analysiert.

**Beispiel 3**

Vorinkubation der RNA

Die Y-Amino-RNA wird in einer Konzentration von 150 - 360 nM in den Test eingesetzt, in der Vorinkubationslösung liegt die Y-Amino-RNA in 2,02-fach höherer Konzentration, in "1xPBS+Mg" (130 mM NaCl, 15,4 mM Na$_2$HPO$_4$, 4,6 mM NaH$_2$PO$_4$, 5 mM MgCl$_2$) und tRNA aus Hefe, 0,3 mg/ml, vor (Volumen: Selektionsrunde 1, 4 ml; ab Selektionsrunde 2, 2 ml; analytischer Ansatz, 300 $\mu$l, siehe unten). Die Vorinkubation erfolgt durch Erhitzen auf 92°C für 1 min und anschließendes Abkühlen auf 37°C innerhalb von 45 min. Die Lösung wird dann direkt weiterverwendet oder zunächst bei -20°C gelagert und vor weiterer Verwendung wieder aufgetaut. Die Vorinkubation wird fortgesetzt durch Inkubation für 20 min bei 37°C; dann wird Cytochalasin B-Lösung (1 mM in Dimethylsulfoxid; Endkonzentration 10 $\mu$M in der Bindungslösung) und eine Erythrozyten-Suspension (Blutkonserve) im Volumenverhältnis 1:1 zugegeben (z.B. 2 ml Vorinkubationslösung mit Cytochalasin B + 2 ml Erythrozyten-Suspension) und vermischt, resultierend in einem Volumen der Bindungslösung von 8 ml (Selektionsrunde 1) bzw. 4 ml (ab Selektionsrunde 2) (analytischer Ansatz, siehe unten), wovon jeweils 1 ml je 75 cm$^2$-Kulturflasche mit HUVEC-Zellen verwendet wird (siehe Beispiel 4). Bis zur weiteren Verwendung wird die Lösung bei 20 bis 30 °C maximal 10 min aufbewahrt, oder bis zu 30 min bei 4°C gelagert. Im analytischen Verfahren (Bindungstest) mit einzelnen RNA-Aptameren wird mit einer Bindungslösung bestehend aus 300 $\mu$l Vorinkubationslösung (mit Cytochalasin B) und 300 $\mu$l Erythrozyten-Suspension gearbeitet, von der Mischung (Bindungslösung 600 $\mu$l) werden zwei Kavitäten mit je 300 $\mu$l beschickt (siehe Beispiel 4).

## Beispiel 4

Selektion von Y-Amino-RNA, und analytischer Bindungstest: Bindung an Zellen und Kompetition durch Antikörper

Eukaryotische Zellen werden nach Standardbedingungen kultiviert, HUVEC-Zellen werden in Kulturflaschen mit 75 cm$^2$ Fläche (Bindungstest analytisch: HUVEC in 6-Loch-Platten) noch drei bis fünf Tage nach Erreichen der Konfluenz weiterkultiviert (siehe Beispiel 7) und dann in den Bindungstest eingesetzt (für jede zu testende Y-Amino-RNA im analytischen Bindungstest werden zwei Kavitäten benötigt, siehe unten). Volumen einer Waschlösung gilt jeweils für eine Kulturflasche, bzw. Kavität; die Zellen werden nach Entfernen des Kulturmediums zweimal mit Serum-freiem Zellkulturmedium (IF-Basalmedium, siehe Beispiel 7; 37°C; Volumen je 5 ml; analytisch: 0,5 ml), dann mit 1xPBS+Mg -Lösung (3 ml; analytisch: 0,4 ml; 18-25°C), dann mit 1xPBS+Mg mit Zusatz von 10 $\mu$M Cytochalasin B gewaschen (3 ml; analytisch: 50 $\mu$l; 18-25°C). Jeweils 1 ml (analytisch: 0,3 ml) der Y-Amino-RNA-Erythrozyten-Suspension (siehe Beispiel 3, "Bindungslösung" nach Vorinkubation) wird nach Entfernen der Waschlösung auf die Zellen gegeben, und die Zellen werden unter Schütteln auf einem Kippschüttler bei einer Frequenz von 30/min und 21 - 30 °C für 60 min inkubiert, wobei alle 10 min die Suspension zusätzlich von Hand durch Kippen der Kulturschale über die Zellen verteilt wird. Nach Zugabe von je 1,25 ml (analytisch: 0,2 ml) Wp3 wird die Suspension entfernt. Die Zellen werden nacheinender mit folgenden Lösungen gewaschen (Temperatur der Waschlösung ist in Klammern angegeben); zweimal mit je 1,5 ml (analytisch: 0,2 ml) Wp3E (4°C); einmal mit je 4 ml (analytisch: 0,5 ml) Wp3EC für 20 min unter Schütteln (siehe oben; bei 21 bis 30 °C); dreimal mit je 2 ml (analytisch: 0,25 ml) Wp3T (21°C); fünf mal mit je 4 ml (analytisch: 0,5 ml) Wp3 (21°C).

Als Antikörper gegen das gewünschte Target IAP (CD47) wurde der anti-CD47-Antikörper "Bric 126" in einer Konzentration von 2 $\mu$g/ml in 1xHBS (25 mM HEPES-NaOH pH 7,4, 0,137 M NaCl, 5 mM KCl, 0,7 mM CaCl$_2$, 0,5 mM MgCl$_2$) und mit 12 $\mu$g/ml Hefe-tRNA angesetzt und davon je 2 ml (analytisch: 0,3 ml, und zusätzlich statt "Bric 126"-, 0,3 ml mit Kontroll-Antikörper Maus IgG) auf die Zellen zur Kompetition der gebundenen Y-Amino-RNA gegeben und 2 Stunden bei 21 - 30 °C unter Schütteln auf einem Kippschüttler (siehe oben) inkubiert, wobei alle 15 min die Lösung zusätzlich durch Kippen der Kulturschale auf den Zellen verteilt wurde. Die Lösung wurde zur Entfernung von Zellen in Eppendorf-Gefäße überführt und 10 min bei 900 - 1000 g und 12-18 °C zentrifugiert. Der Überstand wurde entnommen und mit Ethanol gefällt (siehe Beispiel 1, hier: Fällung für 18-24 Stunden); die Zentrifugation erfolgte bei 20.000 - 21.000 g und 4 - 18 °C, der Niederschlag wurde mit 70 % (v/v) Ethanol p.a. gewaschen, in 50 % (m/v) Guanidinium-isothiocyanat, 25 mM Natriumcitrat, pH 7,0, gelöst, mit einer Mischung aus Phenol und CI (Volumenverhältnis 1:1) extrahiert, die wässrige Phase einer Ethanolfällung unterzogen und zentrifugiert und der Niederschlag gewaschen (siehe Beispiel 1) und dann in 50 - 110 $\mu$l (analytisch: 10 $\mu$l) H$_2$O gelöst; davon wurden 50 - 80 % für die Reverse Transkription eingesetzt (siehe Beispiel 5).

## Beispiel 5

Reverse Transkription der Y-Amino-RNA und Vervielfältigung der DNA-Kopie durch PCR (RT-PCR)

Von den zwei DNA-Primern, die für die PCR zur Synthese der doppelsträngigen DNA verwendet wurden (siehe Beispiel 1), enthielt einer die DNA-Sequenz des T7 RNA Polymerase-Promotors. Der zweite Primer wird für die Reverse Transkription zum Nachweis der RNA durch RT-PCR (Reverse Transkription und anschließende PCR) verwendet. Dazu wird die gereinigte RNA (siehe Beispiel 4) zusammen mit 960 pmol (Selektions-Runde 1) bzw. 240 pmol (ab Selektions-Runde 2, und "analytisch") "primer B" in 110,8 $\mu$l (Sel.-Runde 1), bzw. 27,7 $\mu$l (ab Sel.-Runde 2) (analytisch, 13,9 $\mu$l) inkubiert, in einem Heizblock für 1 min auf 65°C erhitzt und innerhalb von 45 min auf 42°C abkühlen lassen. Dann werden 5-fach konzentrierte Pufferlösung (nach Herstellerangaben für Enzym MMuLV Reverse Transkriptase, "Revert-Aid"), so dass im Ansatz als Endkonzentration 1-fach-Puffer vorliegt, dNTP-Lösung (dATP, dGTP, dTTP und dCTP, jeweils Endkonzentration 1 mM), und 2,4 units/$\mu$l des Enzyms Revert-Aid Reverse Transkriptase (MBI Fermentas) in einem Gesamtvolumen von 210 $\mu$l (Sel.-Runde 1), bzw. 50 $\mu$l (ab Sel.-Runde 2) (analytisch: 25 $\mu$l), zugegeben, vermischt und

30 min bei 42°C und dann 20 min bei 48°C inkubiert. Parallel wird ein Ansatz unter gleichen Konzentrationen aber ohne Zusatz von RNA durchgeführt (Kontrolle "K"), um das unbeabsichtigte Einschleppen von DNA nach der PCR "P1" (siehe unten) nachweisen zu können. Das Enzym wird nach der Reaktion durch Inkubation für 10 min bei 68°C inaktiviert. Dann erfolgt eine Ethanolfällung (mit Waschen des Niederschlags; siehe Beispiel 1), und die Nukleinsäure wird in DEPC-$H_2O$ gelöst und bei -20°C gelagert.

Es werden nachfolgend zwei PCR-Reaktionen ("P1" und "P2") nacheinander durchgeführt, wovon P1 dem Nachweis der Affinität des in Beispiel 4 verwendeten RNA-Pools dient (durch RT-PCR), bzw. im analytischen Ansatz werden spezifisch bindende Aptamere (Beispiel 4, "analytisch") nach der RT-PCR durch eine erhöhte DNA-Menge im Fall des spezifischen Antikörpers (hier Bric 126) im Vergleich zum Kontroll-Antikörper identifiziert (siehe unten, "analytisch"), wobei eine PCR-Reaktion und anschließende Gelelektrophorese durchgeführt wird (siehe Beispiel 6). Ein definierter Anteil (25 - 80 %) der Lösung (nach reverser Transkription) bzw. der Kontroll-Lösung K werden jeweils in einem Volumen von 400 μl (analytischer Ansatz: Volumen 40 μl) unter den vom Hersteller empfohlenen Puffer-Bedingungen (hier: "PCR-Puffer blau", Peqlab, Erlangen) mit dNTP-Lösung (siehe oben, Konzentration jeweils 300 bis 320 μM), den DNA-Primem "primer-A" und "-B", jeweils 320 pmol (analytisch: je 40 pmol) und dem Enzym Taq-Polymerase, 5 bis 15 units (analytisch: 3,5 units), in 500 μl PCR-Eppendorfgefäßen gemischt und eine PCR-Reaktion ("P1") unter folgenden Bedingungen durchgeführt (siehe Beispiel 1): 20 sec 94 °C, dann Zyklus: a) 94 °C, 1 min; 44 °C, 1 min und 30 sec; 72 °C, 1 min und 10 sec; gesamt 25 Zyklen, dann auf 4 °C kühlen, bis weiteres Vorgehen. Nach definierter Zykluszahl, z.B. 6, 9, 12, 15 und 25 Zyklen werden Proben zu je 4 μl entnommen (analytischer Ansatz: nur 18 Zyklen, danach nur eine Probe zu 4 μl), die dann in einer Gelelektrophorese analysiert werden (siehe unten). Die Proben werden entnommen, indem am Ende jedes Zyklus 10 sec vor Ende der 72°C-Phase das Programm unterbrochen wurde. Dabei werden alternativ die Proben ohne Herausnehmen der Probenröhrchen entnommen; oder alle Ansätze werden in ein Eisbad gestellt, die Proben entnommen, die Ansätze zurück in den PCR-Cycler gestellt; anschließend wird das Programm bis zum Ende fortgesetzt. Die Analyse der synthetisierten DNA-Menge nach den angegebenen Zyklen erfolgt nach Trennung der Proben in einem Polyacrylamid-Gel mit anschließender SybrGreen-Färbung (siehe Beispiel 2). An dieser Stelle lässt sich also die Menge der an die Zelle gebundenen Y-Amino-RNA durch quantitative-RT-PCR in jeder Selektionsrunde bestimmen. Wenn sich ein Anstieg der RNA-Menge durch das Erscheinen der PCR-DNA z.B. bereits nach dem PCR-Zyklus 6, anstatt Zyklus 8 oder 9 in früheren Selektionsrunden zeigt, dann muss eine Anreicherung von stärker bindenden Y-Amino-RNAs stattgefunden haben. Nach einer weiteren PCR ("P2") zur Vervielfältigung der DNA erfolgt dann entweder eine weitere Selektionsrunde (Synthese von Y-Amino-RNA, Bindung an Zellen mit Selektion, RT-PCR), oder es werden einzelne Moleküle durch Klonieren, Analyse ihrer Affinität und Sequenzieren der erhaltenen Klone charakterisiert (siehe Beispiel 6). Die präparative PCR ("P2") wird unter ähnlichen Bedingungen wie die PCR "P1" durchgeführt (Primer je 0,8 μM), mit folgenden Unterschieden: 1) in einem Gesamtvolumen von 2 ml, das auf 10 Ansätze zu je 200 μl verteilt wird (analytisch: 150μl); und 2) die Zahl der Zyklen ist auf 12 begrenzt (analytisch: 20 Zyklen). Die Analyse der PCR erfolgt wie oben nach dem letzten Zyklus durch Gelelektrophorese. Nach Extraktion mit Phenol und CI und einer Ethanolfällung der DNA (siehe Beispiel 1) erfolgt die Transkription mit T7 RNA-Polymerase zur Synthese der Y-Amino-RNA (siehe Beispiel 2).

## Beispiel 6

Klonierung und Sequenzierung von DNA-Kopien der selektierten Y-Amino-RNAs und Analyse ihrer Affinität

Die Klonierung von PCR-Fragmenten nach Runde 5 der Selektion von Y-Amino-RNAs und RT-PCR wurde nach bekannten Verfahren durchgeführt (Heyman, J. A., J. Cornthwaite, et al. (1999) Genome Res. 9(4): 383-392; Shuman, S. (1994) J. Biol. Chem. 269(51): 32678-32684), von einzelnen Klonen wurde Plasmid-DNA isoliert (Birnboim, H. C. and J. Doly (1979) Nucleic Acids Res. 7(6): 1513-1523), und mit den Plasmid-DNAs wurden PCR-Reaktionen durchgeführt und danach Y-Amino-RNA synthetisiert, mit denen Bindungstests und funktionelle Tests durchgeführt wurden. Dazu wurde zunächst in zwei Schritten PCR-DNA präpariert (siehe Beispiel 5, entsprechend PCR "P2", analytisch). Erste PCR: Volumen 50 μl; DNA-Matrize, jeweils Plasmid-DNA der einzelnen isolierten Klone (Plasmid-Minipräparation von 1 ml Bakteriensuspension, davon wurden 4 % in die PCR eingesetzt); 18 Zyklen. Zweite PCR: Volumen 150 μl; DNA-Matrize, PCR-DNA aus der ersten PCR, 14 Zyklen. Dann folgte die RNA-Synthese (siehe Beispiel 2, analytische, Vorinkubation (Beispiel 3, "analytisch") und Bindung (Beispiel 4, "analytisch"). Danach folgte die Reverse Transkription (Beispiel 5, "analytisch") und eine PCR, nach folgenden Vorgaben: Entsprechend der Beschreibung in Beispiel 5 ("P1") wurden jeweils PCR-Reaktionen durchgeführt in Reaktionsvolumen von 40 μl, wobei parallel jeweils eine Reaktion mit Proben aus den beiden Kompetitionsreaktionen mit dem Antikörper Bric 126 bzw. dem Kontroll-Antikörper durchgeführt wurden (siehe Besispiel 4). Es wurden 25 PCR-Zyklen durchgeführt, wobei nach 7,10 und 25 Zyklen Proben zu je 4 μl entnommen (Beispiel 5) und durch Gelelektrophorese analysiert wurden (siehe Beispiel 2); eine spezifische Bindung an das Zielprotein CD47 zeigte sich durch mehr erhaltene PCR-DNA nach Kompetition durch den Bric126-Antikörper im Vergleich zum Kontroll-Antikörper. Weiterhin wurden mit den einzelnen RNAs ("Aptamere") funktionelle Tests auf ihre anti-apoptotische Wirkung (siehe Beispiel 7 bis-10) durchgeführt. Einzelne Klone wurden ausgewählt und die zugehörigen Plasmid-DNAs sequenziert. Von der erhaltenen DNA-Sequenz wurde die Sequenz der Y-Amino-RNAs abgeleitet.

**Beispiel 7**

Kultivierung von humanen Endothelzellen aus Nabelschnur-Venen (HUVEC)

Lösungen (steril) :

Kulturmedium: IF-Basalmedium + 15% (v/v) NCS, 5 $\mu$g/ml Transferrin, 5 $\mu$g/ml Heparin, 0,7 $\mu$g/ml FGF, 2 mM L-Glutamin [IF-Basalmedium: 1:1-Mischung aus Iscove's Modifiziertem Dulbecco Medium (IMDM) und Ham's F12, beide von Life Technologies, Paisley (England)]

NCS: Serum neugeborener Kälber (Sebak, Aidenbach)

FGF: Fibroblastenwachstumsfaktor (eigene Herstellung, partiell aufgereinigt aus Schweinehim)

Materialien :

Zellkulturgefäße, gelatiniert

Durchführung:

Die Kultivierung von HUVEC erfolgt in gelatinebeschichteten Kulturgefäßen bei 37°C, 5% CO2 und Wasserdampf-gesättigter Luftatmosphäre. Das Kulturmedium wird alle 2-3 Tage gewechselt; bei Konfluenz werden die Zellen mit einer Teilungsrate von 1:3 bis 1:5 passagiert. HUVEC wachsen streng kontaktinhibiert und bilden einschichtige Zellrasen mit der typischen Kopfsteinpflastermorphologie. Bei Konfluenz erreichen die Kulturen Zelldichten von 4-9 x $10^4$ Zellen/cm$^2$. Für Apoptoseuntersuchungen werden ausschließlich HUVEC-Kulturen der Passagen 1-4 verwendet.

Beschichtung von Kulturgefäßen:

Lösungen (steril) :
Gelatinelösung, 1% (w/v) in Milli-Q-Wasser

1 g Gelatine (zellkulturgetestet) in 100 ml Milli-Q-Wasser suspendieren, durch Autoklavieren für 20 min bei 121°C und 2 bar lösen und bei Raumtemperatur lagern.

PBS (140 mM NaCl, 3 mM KC1, 8 mM $Na_2HPO_4$, 1,5 mM $KH_2PO_4$)

8 g/l NaCl

0,2 g/l KCl

1,44 g/l $Na_2HPO_4$ x 2 H20

0,2 g/l $KH_2PO_4$

Die angesetzte Lösung wird 20 min bei 121°C und 2 bar autoklaviert und bei Raumtemperatur gelagert. Der pH-Wert wird überprüft und liegt zwischen 7,2 und 7,4.

Materialien :

Zellkulturgefäße

Durchführung :

Für die Kultivierung adhärent wachsender Zellen werden Kulturgefäße mit Gelatine beschichtet. Der Boden der Zellkulturgefäße wird mit steriler Gelatinelösung bedeckt, die Zellkulturgefäße werden 15 min bei Raumtemperatur belassen. Die Gelatinelösung wird abgesaugt, die Zellkulturgefäße werden einmal mit PBS gewaschen und können so verwendet werden.

Subkultivierung adhärenter Zellen
Lösungen (steril):
PBS
Trypsin/EDTA (0,05% (w/v)/0,02% (w/v))
0,1 ml Trypsin-Stammlösung
0,05 ml EDTA-Stammlösung

Mit sterilem PBS auf 50 ml auffüllen, und in Portionen zu 10 ml bei -20°C lagern.

Materialien:

Zellkulturgefäße, gelatiniert

Durchführung:

Alle aufgeführten Zelltypen werden mit Trypsin/EDTA-Lösung von der Kulturfläche abgelöst. Das Kulturmedium wird abgesaugt, der Boden des Kulturgefäßes kurz mit PBS gewaschen und mit Trypsin/EDTA-Lösung (~1 ml für eine 25 cm$^2$-Kulturflasche) bedeckt. Die Enzymlösung wird sofort wieder abgesaugt, so dass ein dünner Flüssigkeitsfilm auf den Zellen verbleibt. Die Zellen werden 1-10 min bei Raumtemperatur belassen und das Ablösen der Zellen unter dem Mikroskop verfolgt. Das Ablösen der Zellen kann durch sanftes Aufschlagen des Kulturgefäßes auf der Kante beschleunigt werden. Die Zellen werden in frischem Kulturmedium aufgenommen, eventuell gezählt und in neue Kulturgefäße ausgesät.

**Beispiel 8**

Bestimmung der Apoptoserate durch Färbung apoptotischer Zellen mit DAPI
DAPI gehört zur Indolfarbstoffgruppe und ist ein selektiver DNS-Farbstoff. Der Farbstoff wird bei 340-360 nm angeregt, und das Emissionsmaximum liegt bei 480 nm. Er wird für Apoptoseuntersuchungen eingesetzt [vgl. Cohen et al. (1993) Immunology Today, 14(3), 126-130].

Morphologische Auswertung:

Lösungen:

PBS

Formaldehydlösung

4% (v/v) Formaldehyd in PBS

DAPI-Lösung (Molecular Probes, Leiden, Niederlande)

2 μg/ml DAPI in Methanol

Materialien:

Petrischale (35 mm) oder 24-Loch Platte mit HUVEC-Zellen in Kultur Durchführung:

Der Kulturüberstand einer Petrischale oder 24 Loch Platte wird abgesaugt, der Zellrasen wird 15 Minuten mit 1 ml Formaldehydlösung auf Eis fixiert, zweimal mit 2 ml PBS gewaschen, für 15 Minuten mit 0,5 ml DAPI-Lösung versetzt, mit PBS gewaschen und unter dem Fluoreszenzmikroskop ausgewertet.

Es wird mit dem UV-Filtersatz und einem 20 x oder 40 x Objektiv gearbeitet. 500-1000 Zellen werden zufällig ausgewählt und die Zellen mit apoptotischen Kernen gezählt.

Der Apoptose-Index wird nach folgender Formel berechnet:

$$\text{Apoptose-Index [\%]} = \text{Anzahl apoptotischer Zellen/Gesamtzellzahl x 100}$$

**Beispiel 9**

Testsystem für anti-apoptotisch wirksame Aptamere

Die Zellen werden wie in Beispiel 7 beschrieben kultiviert. Die Zellen werden in die entsprechenden Kulturgefäße (z. B. 24-Lochplatte/0,5 ml pro Kavität) ausgesät und nach dem Erreichen der vollständigen Konfluenz für den eigentlichen Test eingesetzt.

Die Induktion der Apoptose erfolgt durch das von den Endothelzellen selbst produzierte und sekretierte TSP-1, welches sich im Kulturmedium anreichert (Auto-Konditionierung des Kulturmediums).

Es werden Untersuchungen zum Einfluss der verschiedenen Aptamere auf die Apoptoserate von Endothelzellen durchgeführt. Hierfür werden die Aptamere, die in DEPC-behandeltem bidestillierten Wasser gelöst sind (Beispiel 6) in dem Kulturmedium für HUVEC (Beispiel 7) verdünnt und in den angegebenen Konzentrationen eingesetzt. Als Positivkontrolle wird Kulturmedium ohne jegliche Aptamere oder Inhibitoren eingesetzt.

Das Medium mit den Aptameren wird zu den Zellen gegeben und für drei Tage unter Kulturbedingungen (Beispiel 7) inkubiert. Nach 36 Stunden wird das Kulturmedium/ Kulturmedium mit Aptameren einmal gewechselt.

Anschließend werden die Zellen wie in Beispiel 4 beschrieben zur Bestimmung der Apoptoserate mit DAPI gefärbt und der Apoptose-Index nach der angegebenen Formel bestimmt.

Die deutliche Apoptose-induzierende Wirkung des auto-konditionierten Mediums im Falle der Positivkontrolle und die reduzierte Apoptose im Falle der Inhibitionskontrolle zeigen als interne Kontrollen ein Gelingen des Testes an.

**Beispiel 10**

Identifikation von anti-apoptotisch wirksamen Aptameren mit Hilfe des erfindungsgemäßen Verfahrens

Die Zellen werden wie in Beispiel 3 beschrieben kultiviert. Die Zellen werden in die entsprechenden Kulturgefäße (z.B. 24-Lochplatte/0,5 ml pro Kavität) ausgesät und nach dem Erreichen der vollständigen Konfluenz für den Test nach Beispiel 9 eingesetzt. Folgende Proben werden angesetzt:

(K) Kulturmedium [auto-konditioniertes Medium, Basisrate der Apoptose, Kontrolle],

(1) Kulturmedium + Aptamer 89, Konzentration: 150 nM

(2) Kulturmedium + Aptamer 89, Konzentration: 300 nM

(3) Kulturmedium + Aptamer 82, Konzentration: 150 nM

(4) Kulturmedium + Aptamer 82, Konzentration: 300 nM

Nach 72 h Inkubation unter Kulturbedingungen werden die Zellen fixiert, mit DAPI gefärbt und morphologisch unter dem Fluoreszenzmikroskop untersucht. Die apoptotischen Zellen und die Gesamtzellzahl werden bestimmt und der Apoptoseindex berechnet (Prozent apoptotischer Zellen).

Tabelle 1: Es werden folgende Aptamere getestet

| PROBE NO | Aptamer-Bezeichnung, Konzentration | Apoptose -index [%] | Inhibitions -index [%]* |
|---|---|---|---|
| K | Kontrolle | $3,59 \pm 0,54$ | -- |
| (1) | 89, 150 nM | $0,17 \pm 0,15$ | 95,24 |
| (2) | 89, 300 nM | $0,16 \pm 0,14$ | 95,65 |
| (3) | 82,150nM | $1,22 \pm 0,27$ | 66,03 |
| (4) | 82, 300 nM | $0,74 \pm 0,19$ | 79,32 |
| * 100% = keine Apoptose mehr detektierbar; 0% = keine Wirkung = Kontrolle | | | |

**Patentansprüche**

1. Verfahren zur Herstellung von spezifischen Bindemolekülen gegen ausgewählte Zielmoleküle, **dadurch gekennzeichnet, dass**

   a) eine Stoffbibliothek enthaltend potentielle Bindemoleküle mit den Zielmolekülen in Kontakt gebracht wird,

   b) ein unspezifischer Bindungskompetitor zugegeben wird,

   c) mindestens einmal ein Waschvorgang durchgeführt wird,

   d) ein spezifischer Bindungskompetitor für das ausgewählte Zielmolekül in mindestens etwa 10-fach, bevorzugt mindestens etwa 100-fach, besonders bevorzugt mindestens etwa 1000-fach höherer Konzentration als die Dissoziationskonstante KD(M) des Paares aus spezifischem Bindungskompetitor und Zielmolekül in der Stoffbibliothek zugegeben und inkubiert wird, und

   e) die abgelösten spezifischen Bindemoleküle mittels eines für die gewählte Stoffbibliothek geeigneten Verfahrens isoliert und gegebenenfalls amplifiziert werden, und

   f) dass das Zielmolekül in Stufe a) nativ im Verbund mit einer Biomembran, bevorzugt auf der Oberfläche einer Zellmembran oder in der extrazellulären Matrix, und ganz besonders bevorzugt im Verbund mit intakten Zellen vorliegt.

2. Verfahren nach Anspruch 1, **dadurch gekennzeichnet, dass** die Stoffbibliothek eine amplifizierbare Stoffbibliothek ausgewählt aus der Gruppe bestehend aus: Nukleinsäure-Bibliotheken, Phage Display-Bibliotheken, Antikörper-Bibliotheken, Peptid-Bibliotheken oder synthetisch-chemische Bibliotheken ist.

3. Verfahren nach einem der vorstehenden Ansprüche, **dadurch gekennzeichnet, dass** Stufe a) und b) zeitgleich durchgeführt werden.

4. Verfahren nach einem der vorstehenden Ansprüche, **dadurch gekennzeichnet, dass** der unspezifische Kompetitor ausgewählt ist aus der Gruppe bestehend aus: Milchpulver, Serum-Proteine wie Serum-Albumin oder Nukleinsäuren wie tRNA oder Hefe-RNA, Erythrozyten, Erythrozyten- Ghosts (Membran-Hüllen), Zelllinien hematopoetischen Ursprungs, wie Lymphoma- oder Leukämie-Zellen, oder HeLa-Suspensionszellen, sowie Zellorganellen wie Zellkerne oder Mitochondrien, wobei die Zellen, Zellmembranen oder Organellen das Zielmolekül nicht tragen, bevorzugt Erythrozyten, Erythrozyten-Ghosts (Membran-Hüllen), Zelllinien hematopoetischen Ursprungs, wie Lymphoma- oder Leukämie-Zellen, oder HeLa-Suspensionszellen, sowie Zellorganellen wie Zellkerne, Mitochondrien, wobei die Zellen, Zellmembranen oder Organellen das Zielmolekül nicht tragen, und besonders bevorzugt Erythrozyten und/oder Erythrozyten-Ghosts (Membran-Hüllen), die das Zielmolekül nicht tragen.

5. Verfahren nach Anspruch 2, **dadurch gekennzeichnet, dass** die Stufen a) bis e) mehrmals aufeinander folgend, bevorzugt jedoch nicht mehr als drei bis fünfmal durchgeführt werden, wobei ab der zweiten Runde die in Stufe e) erhaltenen Bindemoleküle in Stufe a) anstelle der amplifizierbaren Bibliothek eingesetzt werden.

6. Verfahren nach Anspruch 5, **dadurch gekennzeichnet, dass** die Bibliothek eine Aptamer-Bibliothek ist.

7. Verfahren nach einem der vorstehenden Ansprüche, **dadurch gekennzeichnet, dass** in Stufe a) ein Endozytose-Inhibitor in ausreichender Menge hinzu gegeben wird, um die Endozytose zu inhibieren, bevorzugt Cytochalasin B in einer Menge von 1 bis 10 $\mu$M.

8. Verfahren nach einem der Ansprüche 1 bis 7, **dadurch gekennzeichnet, dass** das ausgewählte Zielmolekül ein membranständiges Molekül ist, von dem auch eine lösliche Form bekannt ist, und in Stufe a) ein Proteinase- Inhibitor zugegeben wird.

9. Verfahren nach einem der vorstehenden Ansprüche, **dadurch gekennzeichnet, dass** der spezifische Bindungskompetitor ausgewählt ist aus der Gruppe bestehend aus Antikörpem und deren Fragmente, Aptamere, Liganden der Zielmoleküle sowie niedermolekulare Kompetitoren.

10. Verfahren nach Anspruch 9, **dadurch gekennzeichnet, dass** der spezifische Bindungskompetitor an ein klinisch oder diagnostisch oder sonst wie interessantes Oberflächenmolekül, bevorzugt IAP oder $\alpha_v\beta_3$ bindet.

11. Kit zur Durchfuhrung des Verfahrens nach Ansprüchen 1-10, umfassend einen unspezifischen Bindungskompetitor und einen Endocytose-Inhibitor.

**Claims**

1. A Method for producing specific binding molecules against selected target molecules, **characterised in that**

   a) a substance library containing potential binding molecules is contacted with the target molecules,
   b) a non-specific binding competitor is added,
   c) at least one washing procedure is carried out,
   d) a specific binding competitor for the selected target molecule at a concentration at least approximately 10 times higher, preferably at least approximately 100 times higher, more preferably at least approximately 1000 times higher, than the dissociation constant KD(M) of the pair consisting of specific binding competitor and target molecule in the substance library is added and incubated, and
   e) the detached specific binding molecule is isolated by means of a method suitable for the selected substance library and is amplified where necessary, and
   f) **in that** the target molecule in step a) is present natively, compounded with a biomembrane, preferably on the surface of a cell membrane or in the extracellular matrix, and most preferably compounded with intact cells.

2. The method according to claim 1, **characterised in that** the substance library is an amplifiable substance library selected from the group consisting of: nucleic acid libraries, phage display libraries, antibody libraries, peptide libraries, and synthetic-chemical libraries.

3. The method according to one of the preceding claims, **characterised in that** steps a) and b) are carried out simultaneously.

4. The method according to one of the preceding claims, **characterised in that** the non-specific competitor is selected from the group consisting of: milk powder, serum proteins such as serum albumin or nucleic acids such as tRNA or yeast RNA, erythrocytes, erythrocyte ghosts (membrane skeletons), cell lines of haematopoietic origin such as lymphoma cells or leukaemia cells, or HeLa suspension cells, as well as cell organelles such as nuclei and mitochondria, wherein the cells, cell membranes or organelles do not carry the target molecule, preferably erythrocytes, erythrocyte ghosts (membrane skeletons), cell lines of haematopoietic origin such as lymphoma cells or leukaemia cells, or HeLa suspension cells, as well as cell organelles such as nuclei and mitochondria, wherein the cells, cell membranes or organelles do not carry the target molecule, and more preferably erythrocytes and/or erythrocyte ghosts (membrane skeletons) which do not carry the target molecule.

5. The method according to claim 2, **characterised in that** steps a) to e) are carried out a number of times in succession, but preferably no more than three to five times, wherein, from the second round onwards, the binding molecules obtained in step e) are used in step a) instead of the amplifiable library.

6. The method according to claim 5, **characterised in that** the library is an aptamer library.

7. The method according to one of the preceding claims, **characterised in that**, in step a), an endocytosis inhibitor is added in sufficient amount in order to inhibit endocytosis, preferably cytochalasin B in an amount of 1 to 10 $\mu$M.

8. The method according to one of claims 1 to 7, **characterised in that** the selected target molecule is a membrane-bound molecule of which a soluble form is also known, and a proteinase inhibitor is added in step a).

9. The method according to one of the preceding claims, **characterised in that** the specific binding competitor is selected from the group consisting of antibodies and their fragments, aptamers, ligands of the target molecules, and low-molecular competitors.

10. The method according to claim 9, **characterised in that** the specific binding competitor binds to a surface molecule of clinical, diagnostic or other interest, preferably IAP or $\alpha_v\beta_3$.

11. A kit for carrying out the method according to claims 1 to 10, comprising a non-specific binding competitor and an endocytosis inhibitor.

**Revendications**

1. Procédé de production de molécules de liaison spécifiques à l'encontre de molécules cibles choisies, **caractérisé par le fait que**

   a) une bibliothèque de substances contenant des molécules de liaison potentielles est mise en contact avec les molécules cibles ;
   b) un compétiteur de liaison non spécifique est ajouté ;
   c) au moins une procédure de lavage est effectuée ;
   d) un compétiteur de liaison spécifique pour la molécule cible choisie à une concentration d'au moins approximativement 10 fois plus, de préférence d'au moins approximativement 100 fois plus, de façon davantage préférée d'au moins approximativement 1000 fois plus, que la constante de dissociation KD(M) de la paire consistant en compétiteur de liaison spécifique et molécule cible dans la bibliothèque de substances est ajouté et incubé ; et
   e) la molécule de liaison spécifique détachée est isolée au moyen d'une méthode appropriée pour la bibliothèque de substances sélectionnée et est amplifiée si nécessaire ; et
   f) **par le fait que** la molécule cible à l'étape a) présente à l'état natif, combinée avec biomembrane, de préférence sur la surface d'une membrane cellulaire ou dans la matrice extracellulaire, et, de la façon que l'on préfère le plus, combinée avec des cellules intactes.

2. Procédé selon la revendication 1, **caractérisé par le fait que** la bibliothèque de substances est une bibliothèque de substances amplifiable choisie dans le groupe consistant en : bibliothèques d'acides nucléiques, bibliothèques d'exposition sur phage, bibliothèques d'anticorps, bibliothèques de peptides et bibliothèques de produits chimiques de synthèse.

3. Procédé selon l'une des revendications précédentes, **caractérisé par le fait que** les étapes a) et b) sont effectuées simultanément.

4. Procédé selon l'une des revendications précédentes, **caractérisé par le fait que** le compétiteur non spécifique est choisi dans le groupe consistant sen : poudre de lait, protéines sériques telles que la sérum albumine ou acides nucléiques tels que l'ARNt ou l'ARN de levure, les érythrocytes, les fantômes d'érythrocytes (squelettes membranaires), les lignées cellulaires d'origine hématopoïétique telles que les cellules de lymphome ou les cellules de leucémie, ou les cellules en suspension HeLa, ainsi que les organelles cellulaires telles que les noyaux et les mitochondries, les cellules, membranes cellulaires ou organelles ne portant pas la molécule cible, de préférence les érythrocytes, les fantômes d'érythrocytes (squelettes membranaires), les lignées cellulaires d'origine hématopoïétique telles que les cellules de lymphome ou les cellules de leucémie, ou les cellules en suspension HeLa, ainsi que les organelles cellulaires telles que les noyaux et les mitochondries, les cellules, membranes cellulaires ou organelles ne portant pas la molécule cible, et, de façon davantage préférée, les érythrocytes et/ou fantômes d'érythrocytes (squelettes membranaires) qui ne portent pas la molécule cible.

5. Procédé selon la revendication 2, **caractérisé par le fait que** les étapes a) à e) sont effectuées un nombre de fois de façon successive, mais de préférence pas plus de trois à cinq fois, où, à partir du second cycle, les molécules de liaison obtenues à l'étape e) sont utilisées à l'étape a) au lieu de la bibliothèque amplifiable.

6. Procédé selon la revendication 5, **caractérisé par le fait que** la bibliothèque est une bibliothèque d'aptamères.

7. Procédé selon l'une des revendications précédentes, **caractérisé par le fait qu'**à l'étape a), un inhibiteur d'endocytose est ajouté en quantité suffisante de façon à inhiber l'endocytose, de préférence la cytochalasine B dans une quantité de 1 à 10 $\mu$M.

8. Procédé selon l'une des revendications 1 à 7, **caractérisé par le fait que** la molécule cible sélectionnée est une molécule liée à la membrane dont une forme soluble est également connue, et un inhibiteur de protéinase est ajouté à l'étape a).

9. Procédé selon l'une des revendications précédentes, **caractérisé par le fait que** le compétiteur de liaison spécifique est choisi dans le groupe consistant en anticorps et leurs fragments, aptamères, ligands des molécules cibles et compétiteurs de faible masse moléculaire.

10. Procédé selon la revendication 9, **caractérisé par le fait que** le compétiteur de liaison spécifique se lie à une molécule de surface d'intérêt clinique, de diagnostic ou autre, de préférence IAP ou $\alpha_v\beta_3$.

11. Coffret pour mettre en oeuvre le procédé selon les revendications 1 à 10, comprenant un compétiteur de liaison non spécifique et un inhibiteur d'endocytose.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5475096 A

**In der Beschreibung aufgeführte Nicht-Patentliteratur**

- **LENZ, G.R. et al.** *Drug Discovery Today,* 2000, vol. 5 (4), 145-156
- **JAYASENA, S. D.** *Clin. Chem.,* 1999, vol. 45 (9), 1628-1650
- **KHANDURINA, J. ; A. GUTTMAN.** *Curr. Opin. Chem. Biol.,* 2002, vol. 6 (3), 359-366
- **MACBEATH, G. ; S. L. SCHREIBER.** *Science,* 2000, vol. 289 (5485), 1760-1763
- **AZRIEL-ROSENFELD, R. et al.** *J. Mol. Biol.,* 2004, vol. 335 (1), 177-192
- **DOUCET, C. ; D. BROUTY-BOYE et al.** *J. Clin. Invest.,* 1998, vol. 101 (10), 2129-2139
- **LARCHE, M. ; D. S. ROBINSON et al.** *J. Allergy Clin. Immunol.,* 2003, vol. 111 (3), 450-463
- **DUDEZ, T. S. ; M. CHANSON et al.** *J. Infect. Dis.,* 2002, vol. 186 (6), 774-781
- **KREIN, P. M. ; B. W. WINSTON.** *Chest,* 2002, vol. 122 (6), 289S-293S
- **SCHMIDT, C. ; T. MARTH et al.** *Pathobiology,* 2002, vol. 70 (3), 177-83
- **MURAKAMI, H. ; S. M. AKBAR et al.** *Clin. Exp. Immunol.,* 2002, vol. 128 (3), 504-510
- **BERLINER, S. ; O. ROGOWSKI et al.** *Cardiology,* 2000, vol. 94 (1), 19-25
- **SCHRAM, M. T. ; N. CHATURVEDI et al.** *Diabetes Care,* 2003, vol. 26 (7), 2165-2173
- **FELDMANN, M et al.** *Cell,* 1996, vol. 85 (3), 307-310
- **DEN BROEDER, A. A. et al.** *Ann. Rheum. Dis.,* 2002, vol. 61 (4), 311-318
- **BASELGA J.** *Eur. J. Cancer,* 2001, vol. 37 (4), S16-S22
- **RIBATTI, D. ; A. VACCA et al.** *Eur. J. Cancer,* 2002, vol. 38 (6), 750-757
- **PECHEUR, I. ; O. PEYRUCHAUD et al.** *Faseb J.,* 2002, vol. 16 (10), 1266-1268
- **FREYBERG, M. A. ; D. KAISER et al.** *Biochem. Biophys. Res. Commun.,* 2001, vol. 286 (1), 141-149
- **LENTSCH, A. B. ; P. A. WARD.** *J. Pathol.,* 2000, vol. 190 (3), 343-348
- **PARKER, C., 3RD ; J. A. VITA et al.** *Atheroslerosis,* 2001, vol. 156 (2), 417-424
- **XU, Q.** *Arteriosler. Thromb. Vasc. Biol.,* 2002, vol. 22 (10), 1547-1559
- **GIRI, R. ; Y. SHEN et al.** *Am. J. Physiol. Cell Physiol.,* 2000, vol. 279 (6), C1772-81
- **JOHNSTONE, M. ; A. J. GEARING et al.** *J. Neuroimmunol.,* 1999, vol. 93 (1-2), 182-193
- **ARAP, W. ; M. G. KOLONIN et al.** *Nat. Med.,* 2002, vol. 8 (2), 121-127
- **BLANK, M. ; T. WEINSCHENK et al.** *J. Biol. Chem.,* 2001, vol. 276 (19), 16464-16468
- **DANIELS, D. A. ; H. CHEN et al.** *Proc. Natl. Acad. Sci. U. S. A.,* 2003, vol. 100 (26), 15416-15421
- **HORNICK, J. L. ; J. SHARIFI et al.** *J. Nucl. Med.,* 2000, vol. 41 (2), 355-362
- **NIELSEN, L. S. ; J. G. HANSEN et al.** *Embo J.,* 1983, vol. 2 (1), 115-119
- **ZHOU, Y. H. ; M. TAKEKOSHI et al.** *Antiviral Res.,* 2002, vol. 56 (1), 51-59
- **RHODES, A. ; A. DEAKIN et al.** *J. Biol. Chem.,* 2000, vol. 275 (37), 28555-28561
- **MARTELL, R. E. ; J. R. NEVINS et al.** *Mol. Ther.,* 2002, vol. 6 (1), 30-34
- **LAMLA, T. ; V. A. ERDMANN.** *J. Mol. Biol.,* 2003, vol. 329 (2), 381-388
- **NORD, K ; O. NORD et al.** *Eur. J. Biochem.,* 2001, vol. 268 (15), 4269-4277
- **HUMMEL, V. ; B. A. KALLMANN et al.** *J. Neuropathol. Exp. Neurol.,* 2001, vol. 60 (4), 320-327
- **MUKAE, H. ; J. ASHITANI et al.** *Respirology,* 2003, vol. 8 (3), 326-331
- **BLACKWELL, H. E. ; L. PEREZ et al.** *Chem. Biol.,* 2001, vol. 8 (12), 1167-1182
- **CLEMONS, P. A. ; A. N. KOEHLER et al.** *Chem. Biol.,* 2001, vol. 8 (12), 1183-1195
- **WOIWODE, T. F. ; J. E. HAGGERTY et al.** *Chem. Biol.,* 2003, vol. 10 (9), 847-858
- **FELLOUSE, F. ; K. DESHAYES.** *Chem. Biol.,* 2003, vol. 10 (9), 783-786
- **FREYBERG et al.** *BBRC,* 2001, vol. 271 (3), 584-588
- **M. HOMANN ; H.U. GÖRINGER.** *Nucleic Acids Res.,* 1999, vol. 27 (9), 2006-2014

- **HEYMAN, J. A. ; J. CORNTHWAITE et al.** *Genome Res.,* 1999, vol. 9 (4), 383-392
- **SHUMAN, S.** *J. Biol. Chem.,* 1994, vol. 269 (51), 32678-32684
- **BIRNBOIM, H. C. ; J. DOLY.** *Nucleic Acids Res.,* 1979, vol. 7 (6), 1513-1523
- **COHEN et al.** *Immunology Today,* 1993, vol. 14 (3), 126-130